# EUROPÄISCHE PATENTSCHRIFT

(11) **EP 1 010 399 B1**
(45) Veröffentlichungstag und Bekanntmachung des Hinweises auf die Patenterteilung: **14.05.2003**
(21) Anmeldenummer: 98121748.2
(22) Anmeldetag: 16.11.1998
(51) Int. Cl.: A61B 19/02

(54) **Sammelbehälter für sterilisierungsbedürftigen Abfall**
Container for medical waste sterilization
Récipient pour la stérilisation de déchets médicaux

(43) Veröffentlichungstag der Anmeldung: 21.06.2000
(73) Patentinhaber: Rigling, Heinz, D-75382 Althengstett (DE)
(72) Erfinder: Rigling, Heinz, D-75382 Althengstett (DE)
(74) Vertreter: Nix, Frank Arnold, Dr.

(56) Entgegenhaltungen:
- EP-A- 0 580 971
- EP-A- 0 875 210
- DE-C- 19 730 472
- US-A- 4 502 606

## Beschreibung

Die vorliegende Erfindung bezieht sich auf einen Sammelbehälter für sterilisierungsbedürftigen Abfall, wie er z.B. in Arztpraxen und Krankenhäusern benutzt wird, um Injektionsnadeln, Wundverbände und dergl. zu sammeln und einer sicheren Entsorgung zuzuführen.

Die Entsorgung von Sammelbehältern mit derartigem Abfall kann durch Verbrennen geschehen. Aus der Druckschrift EP 0 658 334 A1 ist ein aus thermoplastischem Kunststoff bestehender Sammelbehälter bekannt, der aus einem Grundkörper, einem Deckel und einer eine Öffnung im Deckel verschließenden Kappe besteht, wobei das Aufdrücken des Deckels diesen mit dem Grundkörper unlösbar verbindet und die durch die Kappe verschließbare Öffnung als Einwurföffnung zur Verfügung steht. Hier geschieht die Entsorgung durch spezielles Verbrennen des Behälters mit Inhalt, wobei eine vorhergehende Sterilisation natürlich entbehrlich ist.

Wenn eine Entsorgung durch allgemeines Verbrennen (Hausmüllverbrennungsanlage) oder durch Lagern auf einer Hausmülldeponie mit oder ohne vorheriges Schreddern stattfinden soll, erfordert dies ein vorangehendes Sterilisieren des Behälters mit Inhalt. Dies geschieht in Autoklaven, in denen nach Einbringen des Behälters oder mehrerer Behälter zunächst ein Unterdruck erzeugt wird, bevor man überhitzten Dampf (mindestens 105°C, üblich sind 134° C) einströmen läßt, welcher dann alle vorher evakuierten Räume füllt und die Sterilisation bewirkt. Das Evakuieren und Einströmen von Dampf wird gewöhnlich dreimal wiederholt, wobei die gesamte Dauer dieses Vorgangs zwischen 20 und 30 Minuten beträgt.

Ein bekannter, zur Verwendung nach diesem Verfahren vorgesehener Behälter hat eine Sollbruchstelle in der Weise, daß bei Einleiten des Unterdrucks in den Autoklaven unter der Wirkung des dabei auftretenden Druckgefälles von innen nach außen ein Flächenbereich des Behälters unter Freigabe einer Öffnung ausbricht. Durch die entstandene Öffnung kann anschließend der Dampf einströmen.

Nachteilig bei dieser Ausbildung ist, daß das Herausbrechen des Flächenbereichs nicht zuverlässig gewährleistet ist. Es kann vorkommen, daß tatsächlich keine Öffnung entsteht und somit keine Sterilisation des Behälterinhalts stattfindet. Diese Gefahr besteht insbesondere bei Anwendung niedrigerer Dampftemperaturen, die das Material nicht genügend erweichen, um die Sollbruchstelle zu öffnen.

Ein aus EP 0 049 430 A1 bekannter Sammelbehälter für Sondermüll der betrachteten Art besteht insgesamt aus Polystyrol, welches sich bei den im Autoklaven auftretenden Temperaturen zumindest so stark verformt, daß der Deckel abspringt und der Inhalt somit vom eingeleiteten Dampf erreicht wird und die Sterilisation stattfindet. Ein diesem Behälter anhaftender Nachteil ist, daß das stark verformte oder sogar geschmolzene Polystyrol am Boden und an den Wänden des Autoklaven haftet und dessen Reinigung erhebliche Mühe bereitet. Ein weiterer Nachteil ist darin zu sehen, daß der verformte Deckel sich nach dem Sterilisationsvorgang nicht mehr auf den Behälter aufsetzen läßt und dieser im offenen Zustand dem Autoklaven entnommen werden muß und dabei einen unästhetischen Anblick bietet. Wenn der Behälter umgestoßen wird, wird der - wenn auch sterilisierte - Abfall herausfallen und muß wieder eingesammelt werden, was die Gefahr von Schnitt- und Stichverletzungen mit sich bringt.

Aus DE 197 30 472 C1 ist ein Kanülenbehälter bekannt, in dessen Öffnungsverschluß eine bei den im Autoklaven auftretenden Temperaturen schmelzende Platte eingelegt ist. Diese ruht auf einer mit Ventilationsöffnungen versehenen Fläche des Verschlusses. Bei dieser Ausbildung kann das Entstehen einer Öffnung für den Dampfeintritt dadurch verhindert sein, dass die schmelzende Platte die Ventilationsöffnungen verstopft.

Aufgabe der vorliegenden Erfindung ist die Schaffung eines Behälters der betrachteten Art, der einerseits bis zur Sterilisierung zuverlässig geschlossen gehalten werden kann und andererseits im Autoklaven zuverlässig eine Öffnung für den Eintritt des Dampfs freigibt. Diese Öffnung soll dabei verhältnismäßig klein sein, sodaß der Inhalt auf dem Weg zur endgültigen Entsorgung nicht austritt und auch nicht in erheblichem Maße sichtbar wird.

Die Lösung der gestellten Aufgabe gelingt durch die in Patentanspruch 1 angegebenen Merkmale.

Zweckmäßige Weiterbildungen der Erfindung sind in den Unteransprüchen angegeben. Dabei hat der Vorschlag gemäß Anspruch 5 die Bedeutung, daß die Öffnung u.U. schon durch eine Unterdruckeinleitung freigegeben werden kann, wenn der Stopfen unter der Wirkung des auftretenden Druckgefälles abspringt; sofern dies aber nicht geschieht, auf jeden Fall die Öffnung durch das Schrumpfen des Verschlußes freigegeben wird.

Der vorgeschlagene Sammelbehälter wird vorzugsweise einen quadratischen Querschnitt mit abgerundeten Ecken aufweisen, wobei dieser sich von einer Standfläche von 35 x 35 cm konisch nach oben auf die Deckelmaße 40 x 40 cm erweitert. Praxisgeeignete Rauminhalte sind 30 oder 50 1, woraus sich die Höhe des Behälters ergibt. Der Deckel wird mit dem oberen Rand des Behälters auf eine geeignete Weise so verbunden, daß durch das Aufdrücken des Deckels ein Einrasten stattfindet und eine feste, vorzugsweise luftdichte Verbindung zustandekommt.

Die Erfindung wird nachfolgend durch die Beschreibung eines konkreten Ausführungsbeispiels anhand der beigegebenen Zeichnung weiter erläutert. Diese zeigt in perspektivischer Darstellung einen Sammelbehälter mit Dekkel.

Auf den Körper 1 des Sammelbehälters ist ein Deckel 2 aufsetzbar, der längs seines Umfangs Riegelzungen 3 aufweist, welche beim Aufsetzen des Deckels unter den Rand des Behälterkörpers 1 einfallen und so den Deckel mit dem Behälterkörper verrasten. Im Deckel sind Versteifungsrippen 4 und ein Tragegriff 5 ausgeformt.

In einer Ecke des Deckels ist eine Öffnung 6 vorgesehen, die von einem Gitter 7 überdeckt ist und von einem aufragenden Kragen 8 umgeben ist, in welchen ein Verschlußstopfen 9 einsetzbar ist. Dieser besteht aus einem Werkstoff, der bei den im Autoklaven auftretenden Temperaturen schrumpft, während der Werkstoff von Grundkörper 1 und Deckel 2 bei diesen Temperaturen noch beständig ist.

Dem Verbraucher, also insbesondere den Krankenhäusern oder den Arztpraxen, werden die Behälterkörper 1 ineinandergestapelt und die zugehörigen Deckel 2 aufeinandergestapelt mit auf dem Öffnungskragen 8 aufgesetztem Verschlußstopfen 9 zur Verfügung gestellt.

Wenn im Gebrauch ein Behälterkörper mit infektiösem Abfall gefüllt ist, wird der Deckel 2 aufgedrückt, sodaß er am Rand des Behälterkörpers 1 einrastet und dieser luftdicht verschlossen ist. In diesem Zustand kann der Behälter ohne Probleme gelagert und gehandhabt werden, bis er der Sterilisierung zugeführt wird.

Zur Sterilisierung wird der Behälter in den Autoklaven gestellt, wo bei Erreichen der Schrumpftemperatur des Verschlußstopfens 9 dieser schrumpft und dem Dampf den Zutritt freigibt. Eine Möglichkeit ist es, daß der Stopfen 9 in dem öffnungskragen 8 so eingesetzt ist, daß er unter der Wirkung eines Druckunterschieds abspringen kann. Es ist dann möglich, daß schon die Unterdruckeinleitung in den Autoklaven und das dadurch auftretende Druckgefälle von innen nach außen zum Ablösen des Verschlußstopfens führt. Auf jeden Fall aber wird die Öffnung 6 freigegeben, wenn die Schrumpftemperatur des Verschlußstopfens 9 erreicht wird.

Bei der anschließenden Endentsorgung kann der sterilisierte Inhalt des Behälters nicht austreten; dies ist durch das Gitter 7 in der Öffnung 6 verhindert.

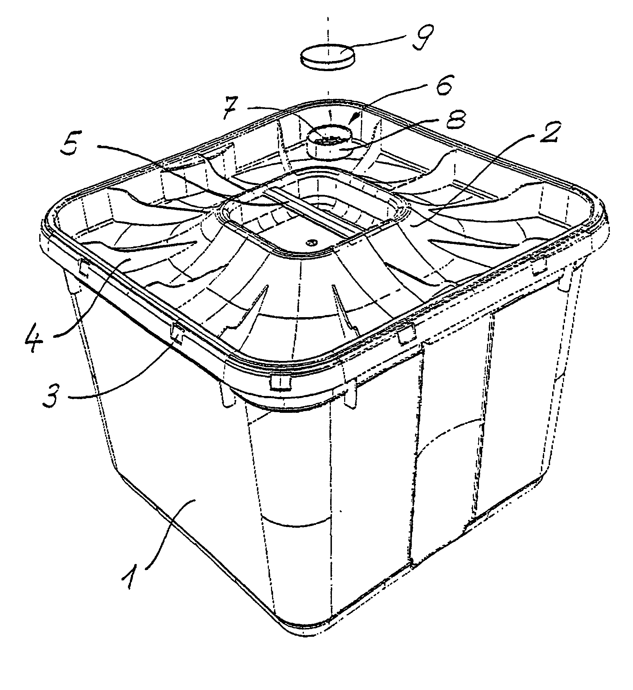

## Patentansprüche

1. Sammelbehälter für sterilisierungsbedürftigen Abfall, der zur Sterilisierung seines Inhalts in einem Autoklaven eine Öffnung (6) mit einem Verschluß aufweist, welcher sich im Autoklaven während der Sterilisierung öffnet um eine Verbindung zwischen dem Behälterinnenraum und der Autoklavenatmosphäre freigibt,
**dadurch gekennzeichnet, daß** der Öffnungsverschluß (9) aus einem Werkstoff besteht, welcher unter der Wirkung der Temperatur im Autoklaven schrumpft.

2. Sammelbehälter nach Anspruch 1, **dadurch gekennzeichnet, daß** der Öffnungsverschluß als ein Stopfen ausgebildet ist, der in einen die Öffnung (6) umgebenden Kragen (8) klemmend eingesetzt ist.

3. Sammelbehälter nach einem oder mehreren der vorhergehenden Ansprüche, **dadurch gekennzeichnet, daß** der Behälter aus einem sich nach oben konisch erweiternden, mehrere ineinander stapelbaren Körper (1) und einem seine obere Öffnung übergreifenden Deckel (2) besteht, und daß die Öffnung (6) im Deckel ausgebildet ist.

4. Sammelbehälter nach einem oder mehreren der vorhergehenden Ansprüche, **dadurch gekennzeichnet, daß** in der Öffnung (6) ein Gitter (7) vorgesehen ist.

5. Sammelbehälter nach einem oder mehreren der vorhergehenden Ansprüche, **dadurch gekennzeichnet, daß** der Verschlußstopfen in der Öffnung derart verrastet ist, daß die Rastverbindung sich unter der Wirkung eines Druckgefälles von innen nach außen lösen kann.

## Claims

1. Container for collecting waste requiring sterilization which for performing the sterilization of its contents comprises an opening (6) with a seal that opens during the sterilization for creating a passage between the interior of the container and the atmosphere of the autoclave,
**characterized in that** the seal (9) of the opening is made of a material which shrinks under the effect of the temperature in the autoclave.

2. container according to claim 1, **characterized in that** the seal of the opening is in the form of a plug wedged into a collar (8) surrounding the opening (6).

3. Container according to one or several of the preceding claims, **characterized in that** the container consists of a body (1) conically diverging towards the top and stackable into each other, and of a cover (2) overlapping its upper opening, and that the opening (6) is formed in the cover.

4. Container according to one or several of the preceding claims, **characterized in that** a grid (7) is provided in the opening (6).

5. Container according to one or several of the preceding claims, **characterized in that** the sealing plug is snapped into the opening in such a way that the locking engagement can disengage under the effect of a pressure drop from inside to outside.

## Revendications

1. Récipient pour déchets exigeant une stérilisation qui, pour la stérilisation de son contenue dans un autoclave, comprend une ouverture (6) avec une fermeture qui s'ouvre pendant la stérilisation et libère un passage entre l'interieur du récipient et l'atmosphère dans l'autoclave,
**caractérisé en ce que** la fermeture (9) se compose d'une matière qui se rétrécit sous l'effet de la temperature dans l'autoclave.

2. Récipient selon la revendication 1, **caractérisé en ce que** la fermeture de l'ouverture est en forme d'un bouchon qui est rangé de façon coinçant dans une collerette (8) entourant l'ouverture (6).

3. Récipient selon une ou plusieurs des revendications précédentes, **caractérisé en ce que** le récipient comprend un corps (1) s'étendant coniquement vers le haut et susceptible d'être empilé l'un dans l'autre et un couvercle (2) chevauchant sa ouverture supérieure, et que l'ouverture (6) est formée dans le couvercle.

4. Récipient selon une ou plusieurs des revendications précédentes, **caracterisé en ce qu'**un treillis (7) est prévu dans l'ouverture (6).

5. Récipient selon une ou plusieurs des revendications précédentes, **caracterisé en ce que** le bouchon est encliqueté dans l'ouverture de telle façon que l'engagement puisse se défaire sous l'effet d'une difference de pression de l'intérieur à l'extérieur.
